# EUROPEAN PATENT APPLICATION

(11) **EP 3 747 495 A1**
(43) Date of publication of application: **09.12.2020**
(21) Application number: 18903767.4
(22) Date of filing: 30.01.2018
(51) Int. Cl.: A61M 25/00

(54) **CATHETER**

(71) Applicant: ASAHI INTECC CO., LTD., Seto-shi, Aichi 489-0071 (JP)
(72) Inventor: KAWASHIMA, Naoya, Seto-shi, Aichi 489-0071 (JP); KUWADA, Shuichi, Seto-shi, Aichi 489-0071 (JP)
(74) Representative: Winter, Brandl, Fürniss, Hübner, Röss, Kaiser, Polte - Partnerschaft mbB
(86) International application number: PCT/JP2018/002949
(87) International publication number: WO 2019/150427

(57) **Abstract**

To provide a catheter including a multilayer body having improved torque transmission force from one end to the other end when the one end is rotated laterally, a multiplayer hollow body, and a multilayer hollow body. A catheter 1, including a catheter main body 3 having an inner layer 7, a braid 5 wound around an outer periphery of the inner layer 7, and an outer layer 9 covering the inner layer 7 and the braid 5, along with a distal tip 2 joined to a distal end of the catheter main body 3, in which the braid 5 includes a plurality of first wires (5a to 5m) wound in one direction and a plurality of second wires (5n to 5z) wound in a direction crossing the one direction, and a distal end joint part 8a joining a distal end of the first first wire 5a and a distal end of the second wire 5n and a distal end joint part 8b joining a distal end of the first wire 5b and a distal end of the second wire 5r are arranged at different positions in the longitudinal direction of the catheter main body 3.

## Description

### TECHNICAL FIELD

The present invention relates to a catheter used by being inserted into a lumen of a blood vessel or the like of a patient.

### BACKGROUND ART

Conventionally, there is known a catheter used by being inserted into a lumen of a blood vessel or the like of a patient. For example, a surgeon performing a procedure (1) inserts a guide wire into the lumen of a catheter, (2) causes the distal end of the guide wire to project from the distal end of the catheter, (3) causes the catheter to reach a lesion by guiding by the guide wire, and (4) places a device such as a stent and an embolic coil at the lesion through the lumen of the catheter having reached the lesion.

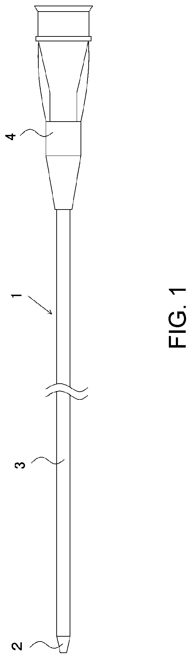

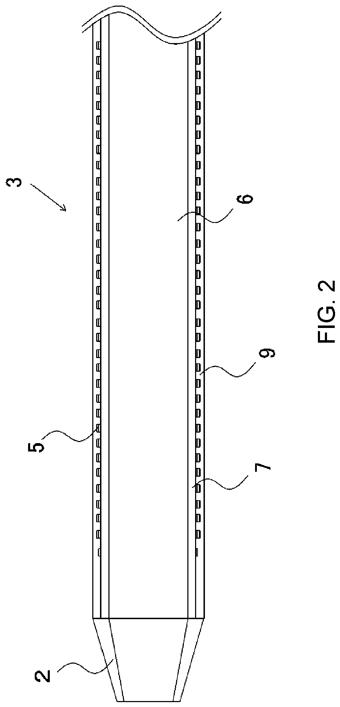

For example, Patent Literature 1 describes a catheter 1 in which a main part 22 is arranged continuously on the proximal end side of a most distal end portion 23 (see Fig. 1 and Fig. 2, for example). The main part 22 includes an inner layer 4, linear bodies 53 to 56 arranged on the outer periphery of the inner layer 4, and an outer layer 6 covering the outer peripheries of the inner layer 4 and the linear bodies 53 to 56 (see Fig. 2 to Fig. 11, for example).

Moreover, Patent Literature 2 describes a catheter 103 including a tubular main body 10 (see Fig. 13, for example). The tubular main body 10 includes an inner layer 11, a linear body 31, and joint members 90 (90a, 90b, 90c, 90d) arranged on the outer periphery of the inner layer 11, and an outer layer 12 covering the outer peripheries of the inner layer 11, the linear body 31, and the joint members 90. The linear body 31 and the joint members 90 are joined through joint parts 92 (see Fig. 13 to Fig. 15, for example).

### CITATION LIST

### Patent Literature

Patent Literature 1: Japanese Patent Application Laid-open No. 2011-72562
Patent Literature 2: Japanese Patent Application Laid-open No. 2012-147956

### SUMMARY OF THE INVENTION

### TECHNICAL PROBLEM

In the above-described conventional catheter, a rigidity difference between the most distal end portion and the linear body of the catheter is large. Therefore, if the conventional catheter is used in the above-described procedure, the distal end portion of the catheter may be kinked due to such a rigidity difference.

Meanwhile, if the linear body is wound around the inner layer of the catheter, particularly if the linear body with high tensile strength is wound around the inner layer in order to improve the torquability and pushability of the catheter, the ends of the linear body may be frayed.

In addition to solving the above-described two problems, it is also necessary to consider production efficiency in winding the linear body around the inner layer of the catheter.

Thus, the present invention aims to provide a catheter capable of preventing the occurrence of a kink at the distal end portion thereof.

The present invention also aims to provide a catheter capable of preventing fraying of the end of the linear body.

Furthermore, the present invention also aims to improve productivity of the catheter.

### SOLUTION TO PROBLEM

A catheter according to one aspect of the invention includes a catheter main body having an inner layer, a braid wound around the outer periphery of the inner layer, and an outer layer covering the inner layer and the braid, and a distal tip joined to a distal end of the catheter main body. The braid includes a plurality of first wires wound in one direction and a plurality of second wires wound in a direction crossing the one direction. Then, a first distal end joint part where a distal end of the first first wire and a distal end of the first second wire are joined and a second distal end joint part where a distal end of the second first wire and a distal end of the second second wire are joined, are arranged at different positions in a longitudinal direction of the catheter main body.

In a preferable embodiment, the second distal end joint part is arranged on a straight line passing the first distal end joint part and being parallel to the axial direction of the catheter.

In a preferable embodiment, the second distal end joint part is arranged at a position separated from a straight line passing the first distal end joint part and being parallel to the axial direction of the catheter.

In a preferable embodiment, a third distal end joint part joining a distal end of the third first wire and a distal end of the third second wire is arranged at a different position in the longitudinal direction of the catheter main body from the first distal end joint part and the second distal end joint part and the position separated from both the straight line passing the first distal end joint part and being parallel to the axial direction of the catheter and the straight line passing the second distal end joint part and being parallel to the axial direction of the catheter.

In a preferable embodiment, the distal ends of all of the first wires are joined to the distal end of any of the second wires or to a point on any second wire other than the distal end.

In a preferable embodiment, the distal ends of all of the second wires are joined to the distal end of any of the first wires or to a point on any first wire other than the distal end.

### BRIEF DESCRIPTION OF DRAWINGS

FIG. 1 is an entire view of a catheter according to a first embodiment of the invention.
FIG. 2 is a longitudinal section view of a distal end portion of the catheter according to the first embodiment.
FIG. 3 is a perspective view of the distal end portion in which an outer layer is removed in the catheter according to the first embodiment.
FIG. 4 is a developed view of the distal end portion in which a braid is developed on a plane surface in the catheter according to the first embodiment.
FIG. 5 is a perspective view of a distal end portion in which an outer layer is removed in a catheter according to a second embodiment.
FIG. 6 is a developed view of the distal end portion in which a braid is developed on a plane surface in the catheter according to the second embodiment.
FIG. 7 is a perspective view of a distal end portion in which an outer layer is removed in a catheter according to a third embodiment.
FIG. 8 is a developed view of the distal end portion in which a braid is developed on a plane surface in the catheter according to the third embodiment.
FIG. 9 is a perspective view of a distal end portion in which an outer layer is removed in a catheter according to a fourth embodiment.
FIG. 10 is a developed view of the distal end portion in which a braid is developed on a plane surface in the catheter according to the fourth embodiment.
FIG. 11 is a perspective view of a distal end portion in which an outer layer is removed in a catheter according to a fifth embodiment.
FIG. 12 is a developed view of the distal end portion in which a braid is developed on a plane surface in the catheter according to the fifth embodiment.

### DESCRIPTION OF EMBODIMENTS

Hereinafter, embodiments of the invention will be described with reference to the enclosed drawings.

### (First Embodiment)

First, the first embodiment of the invention will be described.

FIG. 1 is an entire view of a catheter according to the first embodiment of the invention.

As illustrated in FIG. 1, a catheter 1 of the embodiment includes a catheter main body 3, a distal tip 2 joined to the distal end of the catheter main body 3, and a connector 4 joined to the proximal end of the catheter main body 3.

FIG. 2 is a longitudinal section view of a distal end portion of the catheter according to the first embodiment. The catheter main body 3 includes an inner layer 7, a braid 5 wound around the outer periphery of the inner layer 7, and an outer layer 9 covering the inner layer 7 and the braid 5, as illustrated in FIG. 2.

The inner layer 7 is a long hollow and tubular body made of resin. In the inner layer 7, there is formed a lumen 6 into which a guide wire, other medical devices, and the like are inserted. The resin material forming the inner layer 7 is not particularly limited, and polytetrafluoroethylene (PTFE) is used in the embodiment.

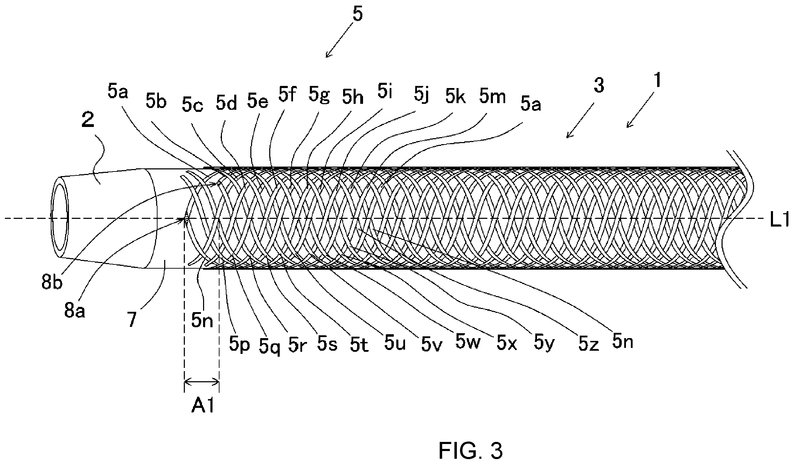

FIG. 3 is a perspective view of the distal end portion in which an outer layer is removed in the catheter according to the first embodiment. As illustrated in FIG. 3, the braid 5 is formed of a total of 24 (12×12) wires including twelve first wires (5a, 5b, 5c, 5d, 5e, 5f, 5g, 5h, 5i, 5j, 5k, and 5m) and twelve second wires (5n, 5p, 5q, 5r, 5s, 5t, 5u, 5v, 5w, 5x, 5y and 5z) that are alternately woven into a mesh form.

In the following, the first wires (5a, 5b, 5c, 5d, 5e, 5f, 5g, 5h, 5i, 5j, 5k, and 5m) are simply referred to as first wires (5a to 5m), and the second wires (5n, 5p, 5q, 5r, 5s, 5t, 5u, 5v, 5w, 5x, 5y, and 5z) are simply referred to as second wires (5n to 5z).

In the embodiment, the twelve first wires (5a to 5m) are wound counterclockwise from the proximal end toward the distal end of the catheter main body 3, as illustrated in FIG. 3. On the other hand, the twelve second wires (5n to 5z) are wound clockwise from the proximal end toward the distal end of the catheter main body 3.

The first wires (5a to 5m) and the second wires (5n to 5z) of the embodiment may be metal wires, and may be stainless steel (SUS304 or SUS316), for example.

In the braid 5 of the embodiment, the distal end of the first wire 5a (first first wire) and the distal end of the second wire 5n (first second wire) are joined to form a distal end joint part 8a (first distal end joint part). Further, the distal end of the first wire 5b (second first wire) and the distal end of the second wire 5r (second second wire) are joined to form a distal end joint part 8b (second distal end joint part).

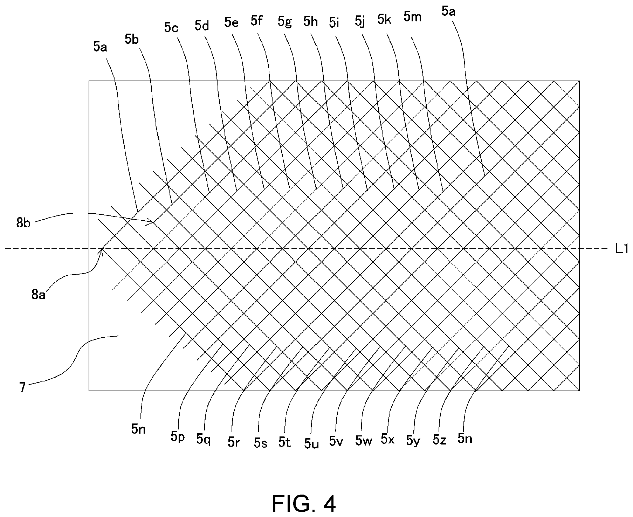

The distal end joint part 8b is located on the more proximal end side in the longitudinal direction of the catheter main body 3 than the distal end joint part 8a. That is, the distal end joint part 8b is located at a different position from the distal end joint part 8a in the longitudinal direction of the catheter main body 3. Here, the different position in the longitudinal direction of the catheter main body 3 indicates that, with a virtual straight line L1 passing the distal end joint part 8a and being parallel to the axial direction of the catheter main body 3 as illustrated in FIG. 3 and FIG. 4 as a reference, the position of the intersection of a perpendicular drawn from the distal end joint part 8b to the virtual straight line L1 is different from the position of the distal end joint part 8a. This also applies to the embodiments described later.

Moreover, the distal end joint part 8b is located at a different position from the distal end joint part 8a in the circumferential direction of the catheter main body 3. That is, with the above-described virtual straight line L1 as a reference, the distal end joint part 8b is arranged not on the virtual straight line L1 but at a point separated from the virtual straight line L1.

FIG. 4 is a developed view of the distal end portion in which the braid 5 is developed on a plane surface in the catheter according to the first embodiment. That is, in FIG. 3, a total of 24 (12×12) wires including 12 first wires (5a to 5m) and 12 second wires (5n to 5z) are alternately woven into a mesh form on the outer surface of the inner layer 7 that is a hollow cylindrical body, while in FIG. 4, such a structure is developed in a plane form for the convenience of explanation.

In FIG. 4, the first wire 5a and the second wire 5n do not exist on the more distal end side than the distal end joint part 8a joining the distal end of the first wire 5a and the distal end of the second wire 5n. That is, the number of wires forming the braid 5 is smaller on the distal end side than on the proximal end side relative to the distal end joint part 8a. This reduces rigidity of the distal end side of the distal end joint part 8a as compared with rigidity of the proximal end side thereof. Further, the distal end of the first wire 5a and the distal end of the second wire 5n are joined, which prevents fraying of the distal end of the first wire 5a and the distal end of the second wire 5n. The prevention of fraying of the first wire 5a and the second wire 5n may also prevent fraying of other wires crossing the first wire 5a or the second wire 5n.

Moreover, the first wire 5b and the second wire 5r do not exist on the more distal end side than the distal end joint part 8b joining the distal end of the first wire 5b and the distal end of the second wire 5r. That is, the number of wires forming the braid 5 is smaller on the distal end side than on the proximal end side relative to the distal end joint part 8b. This reduces rigidity of the distal end side of the distal end joint part 8b as compared with rigidity of the proximal end side thereof. Further, the distal end of the first wire 5b and the distal end of the second wire 5r are joined, which prevents fraying of the distal end of the first wire 5b and the distal end of the second wire 5r. The prevention of fraying of the first wire 5b and the second wire 5r may also prevent fraying of other wires crossing the first wire 5b or the second wire 5r.

When examining the change in rigidity of the catheter main body 3 in the longitudinal direction, the rigidity of an area A1 (see FIG. 3), which is on the more distal end side than the distal end joint part 8b and on the more proximal end side than the distal end joint part 8a, in the catheter main body 3 is reduced as compared with the rigidity of the catheter main body 3 on the more proximal end side than the distal end joint part 8b. This is because the first wire 5b and the second wire 5r do not exist in the area A1, which reduces the rigidity by at least the two wires of the first wire 5b and the second wire 5r.

Moreover, the rigidity of the catheter main body 3 on the more distal end side than the distal end joint part 8a is reduced as compared with the rigidity of the catheter main body 3 on the more proximal end side than the distal end joint part 8b. This is because the first wire 5a, the second wire 5n, the first wire 5b, and the second wire 5r do not exist on the more distal end side than the distal end joint part 8a, which reduces the rigidity by at least these four wires.

That is, in the structures illustrated in FIG. 3 and FIG. 4, the rigidity of the catheter main body 3 is gradually reduced from the proximal end side toward the distal end side. This phenomenon occurs when a plurality of distal end joint parts are located at different positions relative to the longitudinal direction of the catheter main body 3.

Note that in FIG. 4, the distal end portions of the first wires (5c, 5d, 5e, 5f, 5g, 5h, 5i, 5j, 5k, and 5m) other than the first wires 5a and 5b in the embodiment are located on the slightly more distal end side than the intersection with the second wire 5n. This may prevent fraying of the first wires (5c, 5d, 5e, 5f, 5g, 5h, 5i, 5j, 5k, and 5m). Meanwhile, the distal end portions of the first wires (5c, 5d, 5e, 5f, 5g, 5h, 5i, 5j, 5k, and 5m) may extend to the same position (onto the same cross-sectional circumference) as the distal end joint part 8a in the longitudinal direction of the catheter main body 3. In this case, the degree of reduction of rigidity on the distal end side of the catheter main body 3 is suppressed as compared with that of the structure of FIG. 4.

The number of each of the first wires and the second wires of the braid 5 may be other than 12. The combination of the first wires and the second wires is not limited to 12×12 as in the embodiment. It may be, for example, a symmetrical combination such as 8×8 or 4×4, or an asymmetric combination such as 12×8 or 8×4.

Further, the wire width of the first wires (5a to 5m) and the wire width of the second wires (5n to 5z) may be same. Alternatively, the width of one of the wires may be larger than the width of the other wires.

Further, while the material of the first wires (5a to 5m) and the second wires (5n to 5z) of the embodiment is stainless steel (SUS304 or SUS316) as described above, it is possible to use a metal other than stainless steel (platinum, tungsten, or the like, for example) or a material other than metal (reinforced plastic, for example).

Further, the first wires (5a to 5m) and the second wires (5n to 5z) of the embodiment may be made of the same material or made of different materials.

In the embodiment, the first wires (5a to 5m) and the second wires (5n to 5z) are wires having a rectangular cross section (so-called flat wires). However, it is possible to use wires having other cross-sectional shapes such as a round cross section (so-called round wires), for example.

One of the first wires (5a to 5m) and the second wires (5n to 5z) may be round wires, and the other may be flat wires. For example, the first wires (5a to 5m) may be round wires, and the second wires (5n to 5z) may be flat wires. Conversely, the first wire (5a to 5m) may be flat wires, and the second wires (5n to 5z) may be round wires.

The outer layer 9 is made of resin and covers the inner layer 7 and the braid 5. The resin material forming the outer layer 9 is not particularly limited, and may be, for example, polyamide, polyamide elastomer, polyester, or polyurethane. In the embodiment, polyamide is used.

Meanwhile, the distal tip 2 provided at the distal end of the catheter main body 3 has a cylindrical shape with a lumen communicated to the lumen 6 of the catheter main body 3. The distal tip 2 has higher flexibility than the catheter main body 3. The distal tip 2 has a tapered outer peripheral surface gradually shrinking in diameter toward the most distal end portion.

The resin material forming the distal tip 2 is not particularly limited, and it is possible to use a resin material that is more flexible than the resin material forming the inner layer 7 and the outer layer 9. As such a resin material, a polyurethane elastomer is used in the embodiment.

In the embodiment, tungsten powder is contained in the distal tip 2. This improves the visibility of the distal tip 2 under X-ray fluoroscopy.

The connector 4 is made of resin, has a lumen communicated to the lumen 6 of the catheter main body 3, and is joined to the proximal end of the catheter main body 3.

The catheter 1 of the embodiment includes the catheter main body 3 having the inner layer 7, the braid 5 wound around the outer periphery of the inner layer 7, and the outer layer 9 covering the inner layer 7 and the braid 5, along with the distal tip 2 joined to the distal end of the catheter main body 3. The braid 5 includes the first wires (5a to 5m) wound in one direction and the second wires (5n to 5z) wound in a direction crossing the one direction. Then, the distal end joint part 8a joining the distal end of the first wire 5a and the distal end of the second wire 5n and the distal end joint part 8b joining the distal end of the first wire 5b and the distal end of the second wire 5r are located at different positions in the longitudinal direction of the catheter main body 3. The braid 5 is not arranged in an area between the distal end joint part 8a and the distal end of the catheter main body 3. This reduces the number of wires forming the braid 5 toward the distal end of the catheter main body 3 and reduces the rigidity of the catheter main body 3 accordingly. That is, a rigidity difference between the highly flexible distal tip 2 and the catheter main body 3 is reduced, which suppresses kinking of the catheter 1. Further, the distal end joint parts 8a and 8b prevent fraying of the first wire 5a, the first wire 5b, the second wire 5n, and the second wire 5r, and also prevent fraying of other wires crossing the first wire 5a, the first wire 5b, the second wire 5n, and the second wire 5r.

### (Second Embodiment)

Next, the second embodiment of the invention will be described.

A catheter 10 of the second embodiment is different from the catheter 1 of the first embodiment in the number of distal end joint parts. That is, the catheter 1 of the first embodiment includes two distal end joint parts 8a, 8b, while the catheter 10 of the second embodiment includes three distal end joint parts.

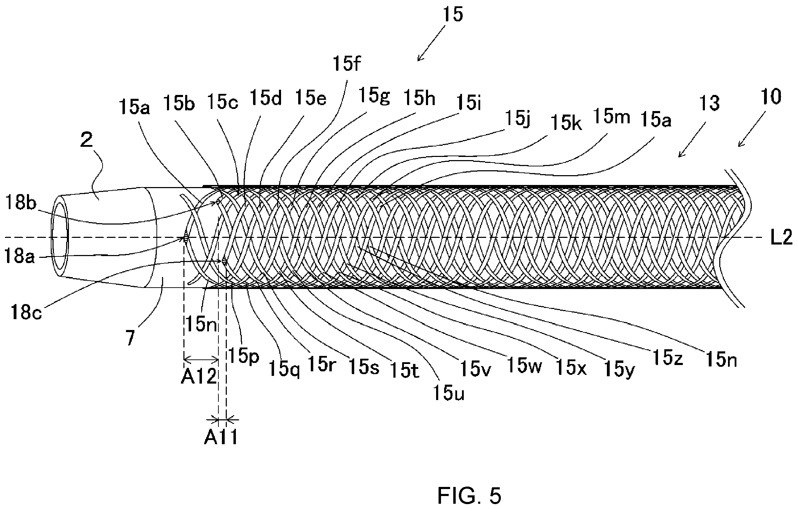

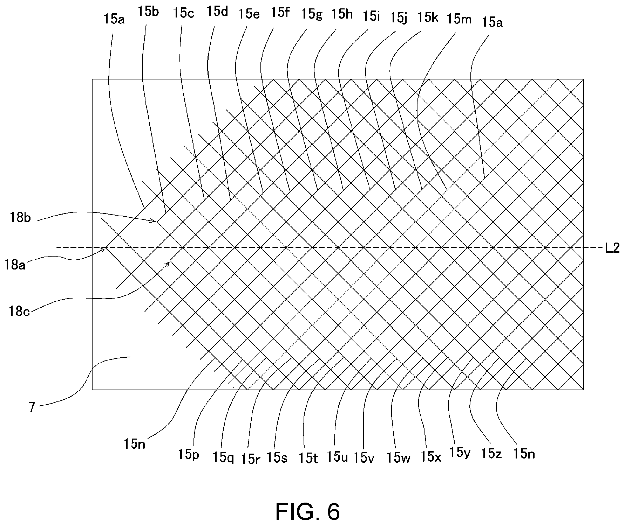

FIG. 5 is a perspective view of the distal end portion in which the outer layer is removed in the catheter according to the second embodiment. FIG. 6 is a developed view of the distal end portion in which the braid is developed on a plane surface in the catheter according to the second embodiment.

Note that the entire view of the catheter is the same as FIG. 1, and the longitudinal section view of the distal end portion of the catheter is also the same as FIG. 2. Thus, the description thereof is omitted. Moreover, in the catheter 10 of the embodiment, a catheter main body 13 is different from the catheter main body 3 of the first embodiment. To be more specific, a braid 15 is different from the braid 5 of the first embodiment. Other structures are the same as those of the first embodiment. Therefore, the same parts are denoted by the same reference numerals, and descriptions thereof may be omitted.

Similarly to the first embodiment, the braid 15 is formed of a total of 24 (12×12) wires including twelve first wires (15a, 15b, 15c, 15d, 15e, 15f, 15g, 15h, 15i, 15j, 15k, and 15m, 15a to 15m) and twelve second wires (15n, 15p, 15q, 15r, 15s, 15t, 15u, 15v, 15w, 15x, 15y and 15z, 15n to 15z) that are alternately woven into a mesh form.

In the embodiment, the twelve first wires (15a to 15m) are wound counterclockwise from the proximal end toward the distal end of the catheter main body 13, as illustrated in FIG. 5. The twelve second wires (15n to 15z) are wound clockwise from the proximal end toward the distal end of the catheter main body 13.

The first wires (15a to 15m) and the second wires (15n to 15z) of the embodiment are also made of metal wires similarly to the first embodiment, and are made of stainless steel (SUS304 or SUS316), for example.

In the braid 15 of the embodiment, the distal end of the first wire 15a (first first wire) and the distal end of the second wire 15n (first second wire) are joined to form a distal end joint part 18a (first distal end joint part). Moreover, the distal end of the first wire 15b (second first wire) and the distal end of the second wire 15r (second second wire) are joined to form a distal end joint part 18b (second distal end joint part). Further, the distal end of the first wire 15d (third first wire) and the distal end of the second wire 15q (third second wire) are joined to form a distal end joint part 18c (third distal end joint part).

The distal end joint part 18b is located on the more proximal end side of the catheter main body 13 than the distal end joint part 18a. That is, the distal end joint part 18b is located at a different position from the distal end joint part 18a in the longitudinal direction of the catheter main body 13. The distal end joint part 18c is located on the more proximal end side of the catheter main body 13 than the distal end joint part 18b. That is, the distal end joint part 18c is located at a position different from both the distal end joint part 18a and the distal end joint part 18b in the longitudinal direction of the catheter main body 13. That is, with a virtual straight line L2 passing the distal end joint part 18a and being parallel to the axial direction of the catheter main body 13 as illustrated in FIG. 5 and FIG. 6 as a reference, the positions of the intersections of perpendiculars drawn from the distal end joint part 18b and the distal end joint part 18c to the virtual straight line L2 are both different from the position of the distal end joint part 18a.

Moreover, the distal end joint part 18a, the distal end joint part 18b, and the distal end joint part 18c are located at mutually different positions in the circumferential direction of the catheter main body 3. That is, with the above-described virtual straight line L2 as a reference, the distal end joint part 8b is arranged not on the virtual straight line L2 but at a point separated from the virtual straight line L2.

In FIG. 6, the first wire 15a and the second wire 15n do not exist on the more distal end side than the distal end joint part 18a. Thus, the rigidity of the more distal end side than the distal end joint part 18a is reduced as compared with the rigidity of the more proximal end side than the distal end joint part 18a, similarly to the first embodiment. Further, the distal end of the first wire 15a and the distal end of the second wire 15n are joined, which prevents fraying of the first wire 15a and the second wire 15n, similarly to the first embodiment. Furthermore, it is possible to prevent fraying of other wires crossing the first wire 15a or the second wire 15n.

The distal end joint part 18b and the distal end joint part 18c also exert the same above-described effects as the distal end joint part 18a. That is, regarding the effect of reducing rigidity, the rigidity of the more distal end side than the distal end joint part 18b and the distal end joint part 18c is reduced as compared with the rigidity of the more proximal end side than the distal end joint parts. Also, regarding the effect of preventing fraying, the distal end joint part 18b prevents fraying of the first wire 15b and the second wire 15r, and the distal end joint part 18c prevents fraying of the first wire 15d and the second wire 15q. Further, the distal end joint part 18b also prevents fraying of other wires crossing the first wire 15b or the second wire 15r, and the distal end joint part 18c also prevents other wires crossing the first wire 15d or the second wire 15q.

When examining the change in the longitudinal rigidity of the catheter main body 13, the rigidity of the catheter main body 13 is gradually reduced from the proximal end side toward the distal end side. This is because in an area A11 (see FIG. 5), which is on the more distal end side than the distal end joint part 18c and on the more proximal end side than the distal end joint part 18b, in the catheter main body 13, the number of wires is smaller by two as compared with the more proximal end side than the distal end joint part 18c in the catheter main body 13, as described above. Thus, the rigidity is reduced accordingly. Similarly, in an area A12 (see FIG. 5), which is on the more distal end side than the distal end joint part 18b and on the more proximal end side than the distal end joint part 18a, in the catheter main body 13, the number of wires is further smaller by two and is smaller by four as compared with the catheter main body 13 on the proximal end side than the distal end joint part 18c. Furthermore, in the catheter main body 13 on the more distal end side than the distal end joint part 18a, the number of wires is still further smaller by two and is smaller by six as compared with the catheter main body 13 on the proximal end side than the distal end joint part 18c. That is, as compared with the catheter 1 of the first embodiment, it is recognized that, in the catheter 10 of the embodiment, the degree of reduction in rigidity is larger toward the distal end because the number of distal end joint parts is increased.

In FIG. 6, the distal end portions of the first wires (15c, 15e, 15f, 15g, 15h, 15i, 15j, 15k, and 15m) whose distal ends are not joined are located on the slightly more distal end side than the intersection with the second wire 15n. This may prevent fraying of the first wires (15c, 15d, 15e, 15f, 15g, 15h, 15i, 15j, 15k, and 15m). Meanwhile, the distal end portions of the first wires (15c, 15d, 15e, 15f, 15g, 15h, 15i, 15j, 15k, and 15m) may extend to the same position (onto the same cross-sectional circumference) as the distal end joint part 18a in the longitudinal direction of the catheter main body 13. In this case, the degree of reduction of rigidity on the distal end side of the catheter main body 13 is suppressed as compared with that of the structure of FIG. 6.

Note that the number of each of the first wires (15a to 15m) and the second wires (15n to 15z) and the combination thereof, the wire width, the material, the cross-sectional shape, and the like may be changed variously as described in the first embodiment.

Furthermore, the embodiment includes three distal end joint parts 18a, 18b, and 8c. However, the number of the distal end joint parts is not limited thereto. For example, in the braid 15 including twelve first wires (15a to 15m) and twelve second wires (15n to 15z), it is possible to form one to twelve distal end joint parts at different positions in the longitudinal direction of the catheter main body 13.

In general, in a braid including N pieces of first wires and N pieces of second wires, it is possible to form the maximum of N pieces of distal end joint parts at different positions in the longitudinal direction of the catheter main body. This point can be easily understood from the arrangement state of the two distal end joint parts in the first embodiment and the three distal end joint parts in the second embodiment.

The catheter 10 of the embodiment includes the catheter main body 13 having the inner layer 7, the braid 15 wound around the outer periphery of the inner layer 7, and the outer layer 9 covering the inner layer 7 and the braid 15, along with the distal tip 2 joined to the distal end of the catheter main body 13. The braid 15 includes the first wires (15a to 15m) wound in one direction and the second wires (15n to 15z) wound in a direction crossing the one direction. Then, the distal end joint part 18a joining the distal end of the first wire 15a and the distal end of the second wire 15n, the distal end joint part 18b joining the distal end of the first wire 15b and the distal end of the second wire 15r, and the distal end joint part 18c joining the distal end of the first wire 15d and the distal end of the second wire 15q are located at different positions in the longitudinal direction of the catheter main body 13. The braid 15 is not arranged in an area between the distal end joint part 18a and the distal end of the catheter main body 13. This reduces the number of wires forming the braid 15 toward the distal end of the catheter main body 13, and reduces the rigidity of the catheter main body 13 accordingly. That is, a rigidity difference between the highly flexible distal tip 2 and the catheter main body 13 is reduced, which suppresses kinking of the catheter 10. As compared with the first embodiment, the number of the distal end joint parts is larger. Thus, the number of reduced wires is also larger toward the distal end. As a result, the degree of reduction in rigidity is larger than that of the first embodiment. Further, it is possible to prevent fraying of the first wire 15a, the first wire 15b, the first wire 15d, the second wire 15n, the second wire 15r, and the second wire 15q, and also prevent fraying of other wires crossing such six wires.

### (Third Embodiment)

Next, the third embodiment of the invention will be described.

A catheter 20 of the third embodiment is different from the catheter 1 of the first embodiment in the positions of the distal end joint parts.

That is, the distal end joint part 8a and the distal end joint part 8b of the catheter 1 of the first embodiment are in a diagonal positional relationship, while four distal end joint parts of the catheter 20 in the third embodiment are arranged on a straight line along the longitudinal direction of a catheter main body 23.

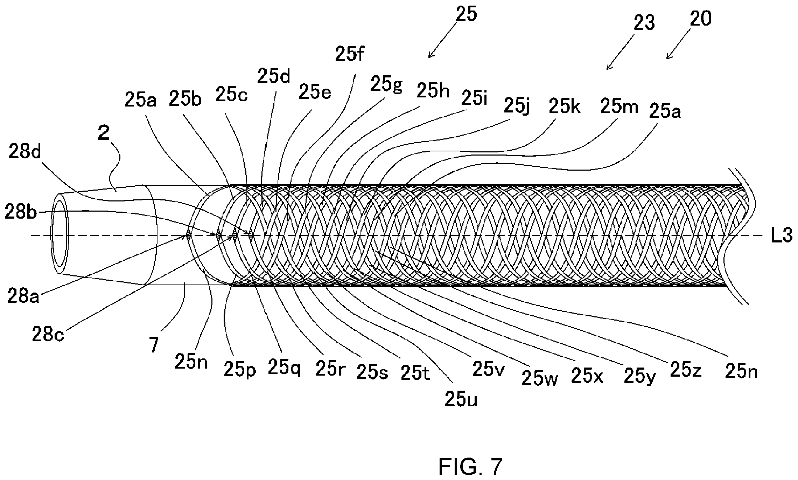

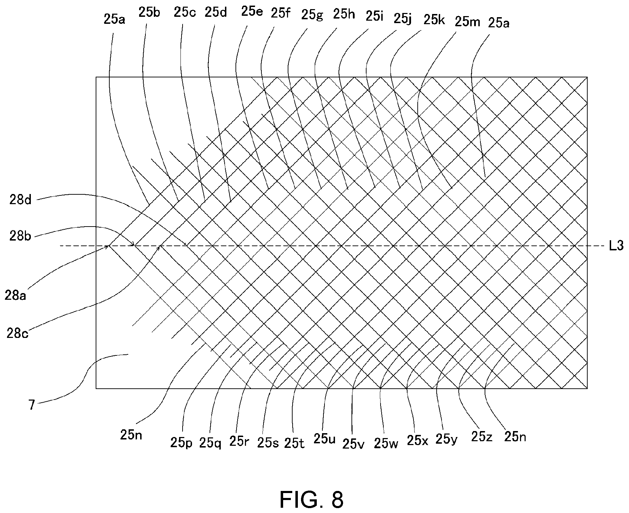

FIG. 7 is a perspective view of a distal end portion in which the outer layer is removed in the catheter according to the third embodiment. FIG. 8 is a developed view of the distal end portion, in which a braid is developed on a plane surface in the catheter according to the third embodiment.

Note that the entire view of the catheter is the same as FIG. 1, and the longitudinal section view of the distal end portion of the catheter is also the same as FIG. 2. Thus, the description thereof is omitted. Moreover, in the catheter 20 of the embodiment, a catheter main body 23 is different from the catheter main body 3 of the first embodiment. To be more specific, a braid 25 is different from the braid 5 of the first embodiment. Other structures are the same as those of the first embodiment. Therefore, the same parts are denoted by the same reference numerals, and descriptions thereof may be omitted.

Similarly to the first embodiment, the braid 25 is formed of a total of 24 (12×12) wires including twelve first wires (25a, 25b, 25c, 25d, 25e, 25f, 25g, 25h, 25i, 25j, 25k, and 25m, 25a to 25m) and twelve second wires (25n, 25p, 25q, 25r, 25s, 25t, 25u, 25v, 25w, 25x, 25y and 25z, 25n to 25z) that are alternately woven into a mesh form.

In the embodiment, as illustrated in FIG. 7, the twelve first wires (25a to 25m) are wound counterclockwise from the proximal end toward the distal end of the catheter main body 23, and the twelve second wires (25n to 25z) are wound clockwise from the proximal end toward the distal end of the catheter main body 23.

The first wires (25a to 25m) and the second wires (25n to 25z) of the embodiment may be metal wires, similarly to the first embodiment, and may be stainless steel (SUS304 or SUS316), for example.

In the braid 25 of the embodiment, four distal end joint parts 28a to 28d are formed. That is, a distal end joint part 28a joining the distal end of the first wire 25a and the distal end of the second wire 25n, a distal end joint part 28b joining the distal end of the first wire 25b and the distal end of the second wire 25p, a distal end joint part 28c joining the distal end of the first wire 25c and the distal end of the second wire 25q, and a distal end joint part 28d joining the distal end of the first wire 25d and the distal end of the second wire 25r are each formed.

In addition, as illustrated in FIG. 7, the distal end joint part 28a, the distal end joint part 28b, the distal end joint part 28c, and the distal end joint part 28d are arranged at different positions along the longitudinal direction of the catheter main body 23. In other words, the distal end joint part 28a, the distal end joint part 28b, the distal end joint part 28c, and the distal end joint part 28d are arranged at mutually different positions in the longitudinal direction. Further, the distal end joint part 28a, the distal end joint part 28b, the distal end joint part 28c, and the distal end joint part 28d are all positioned on a virtual straight line L3 (see FIG. 7 and FIG. 8) parallel to the axial direction of the catheter main body 23. In the embodiment, the four distal end joint parts 28a to 28d exist on the same straight line. However, the number of distal end joint parts positioned on the same straight line may be an arbitrary number equal to or greater than two.

In FIG. 8, the first wire 25a and the second wire 25n do not exist on the more distal end side than the distal end joint part 28a. Thus, the rigidity of the catheter main body 23 on the more distal end side than the distal end joint part 28a is reduced as compared with the rigidity thereof on the more proximal end side than the distal end joint part 28a, similarly to the first embodiment. Further, the distal end of the first wire 25a and the distal end of the second wire 25n are joined, which prevents fraying of the first wire 25a and the second wire 25n, similarly to the first embodiment. Furthermore, it is also possible to prevent fraying of other wires crossing the first wire 25a or the second wire 25n.

The distal end joint part 28b, the distal end joint part 28c, and the distal end joint part 28d also exert the same above-described effects as the distal end joint part 28a. That is, regarding the effect of reducing rigidity, the rigidity on the more distal end side than the distal end joint part 28b, the distal end joint part 28c, and the distal end joint part 28d is reduced as compared with the rigidity of the proximal end side thereof. Also, regarding the effect of preventing fraying, the distal end joint part 28b prevents fraying of the first wire 25b and the second wire 25p, the distal end joint part 28c prevents fraying of the first wire 25c and the second wire 25q, and the distal end joint part 28d prevents fraying of the first wire 25d and the second wire 25r. Further, the distal end joint part 28b also prevents fraying of other wires crossing the first wire 25b or the second wire 25p, the distal end joint part 28c also prevents fraying of other wires crossing the first wire 25c or the second wire 25q, and the distal end joint part 28d also prevents fraying of other wires crossing the first wire 25d or the second wire 25r.

When examining the change in the longitudinal rigidity of the catheter main body 23, the rigidity of the catheter main body 23 is gradually reduced from the proximal end side toward the distal end side, similarly to the second embodiment. This is because in the catheter main body 23 on the more distal end side than the distal end joint part 28d, the number of wires is smaller by two as compared with the catheter main body 23 on the more proximal end side than the distal end joint part 28d. Thus, the rigidity is reduced accordingly. Further, the same applies to the distal end joint parts 28c, 28b, and 28a. As compared with the catheters 1, 10 of the first and second embodiments, it is recognized that, in the catheter 20 of the embodiment, the degree of reduction in rigidity is larger toward the distal end because the number of the distal end joint parts is increased.

In addition, the distal end joint part 28a, the distal end joint part 28b, the distal end joint part 28c, and the distal end joint part 28d are arranged at different positions on the same straight line along the longitudinal direction of the catheter main body 23. This allows for easy manufacturing. For example, a laser welding machine is linearly moved in the axial direction relative to the catheter 20 to weld wires at a plurality of locations, enabling to join the wires efficiently.

In FIG. 8, the distal end portions of the first wires (25e, 25f, 25g, 25h, 25i, 25j, 25k, and 25m) whose distal ends are not joined are located on the slightly more distal end side than the intersections with the second wires. Similarly, the distal end portions of the second wires (25s, 25t, 25u, 25v, 25w, 25x, 25y, and 25z) whose distal ends are not joined are located on the slightly more distal end side than the intersections with the first wires. However, the distal end portion of each of these wires may extend to the same position as the distal end joint part 28a in the longitudinal direction of the catheter main body 23. In this case, the degree of reduction of rigidity on the distal end side of the catheter main body 23 is suppressed as compared with that of the structure of FIG. 8.

Note that the number of each of the first wires (25a to 25m) and the second wires (25n to 25z), the combination thereof, the wire width, the material, the cross-sectional shape, and the like may be changed variously as described in the first embodiment.

Furthermore, the embodiment includes four distal end joint parts 28a, 28b, 28c, and 28d. However, the number of the distal end joint parts is not limited thereto. For example, in the braid 25 including twelve first wires and twelve second wires, it is possible to form two to twelve distal end joint parts that are arranged at different positions on the same straight line in the longitudinal direction of the catheter main body 23.

In general, in a braid including N pieces of first wires and N pieces of second wires, it is possible to form the maximum of N pieces of distal end joint parts at different positions along the longitudinal direction of the catheter main body. It is possible to analogize this point from FIG. 8.

In the catheter 20 of the embodiment, the distal end joint part 28a, the distal end joint part 28b, the distal end joint part 28c, and the distal end joint part 28d are arranged on a straight line parallel to the axial direction of the catheter main body 23. Thus, it is possible to improve manufacturing efficiency, prevent fraying of the braid 25, reduce a rigidity difference between the distal tip 2 and the braid 25, and prevent kinking of the catheter 20.

### (Fourth Embodiment)

Next, the fourth embodiment of the invention will be described. A catheter 30 of the fourth embodiment is different from the catheter 20 of the third embodiment in the processing method of the distal end of the second wire.

That is, in the second wires of the catheter 20 of the third embodiment, the distal end of each of the four seconds wires (25n, 25p, 25q, and 25r) is joined to the distal end of each of the first wires (25a, 25b, 25c, and 25d), while in the second wires of the catheter 30 of the embodiment, the distal ends of all of the second wires (35n, 35p, 35q, 35r, 35s, 35t, 35u, 35v, 35w, 35x, 35y, and 35z) are joined to a distal end of any of the first wires or to a point on the first wires other than the distal ends.

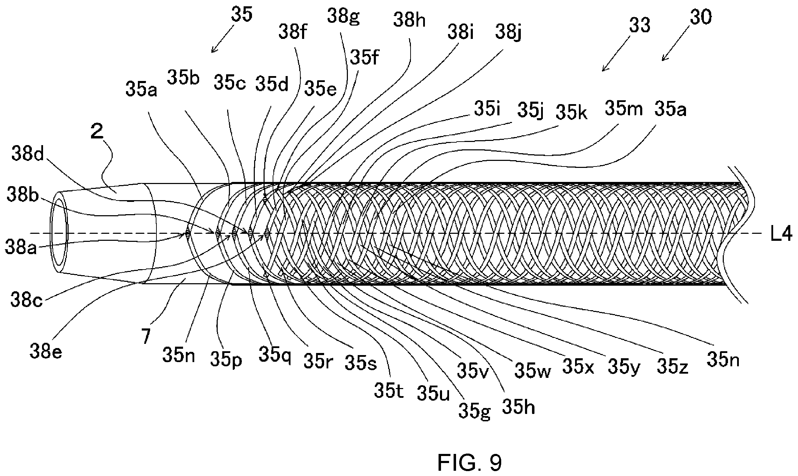

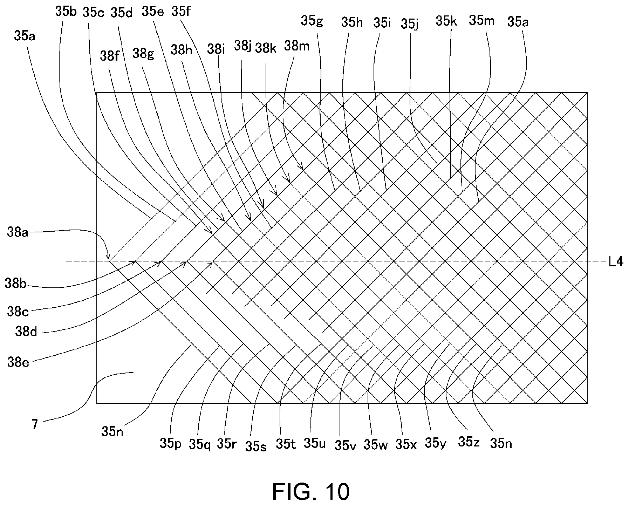

FIG. 9 is a perspective view of the distal end portion in which the outer layer is removed in the catheter according to the fourth embodiment, and FIG. 10 is a developed view of the distal end portion in which the braid is developed on a plane surface in the catheter according to the fourth embodiment.

Note that the entire view of the catheter is the same as FIG. 1, and the longitudinal section view of the distal end portion of the catheter is also the same as FIG. 2. Thus, the description thereof is omitted. Moreover, in the catheter 30 of the embodiment, a catheter main body 33 is different from the catheter main body 3 of the first embodiment. To be more specific, a braid 35 is different from the braid 5 of the first embodiment. Other structures are the same as those of the first embodiment. Therefore, the same parts are denoted by the same reference numerals, and descriptions thereof may be omitted.

Similarly to the first embodiment, the braid 35 is formed of a total of 24 (12×12) wires including twelve first wires (35a, 35b, 35c, 35d, 35e, 35f, 35g, 35h, 35i, 35j, 35k, and 35m, 35a to 35m) and twelve second wires (35n, 35p, 35q, 35r, 35s, 35t, 35u, 35v, 35w, 35x, 35y and 35z, 35n to 35z) that are alternately woven into a mesh form.

In the embodiment, as illustrated in FIG. 9, the twelve first wires (35a to 35m) are wound counterclockwise from the proximal end toward the distal end of the catheter main body 33, and the twelve second wires (35n to 35z) are wound clockwise from the proximal end toward the distal end of the catheter main body 33.

The first wires (35a to 35m) and the second wires (35n to 35z) of the embodiment may be metal wires, similarly to the first embodiment, and may be stainless steel (SUS304 or SUS316), for example.

In the braid 35 of the embodiment, five distal end joint parts 38a to 38e are formed. That is, the distal end joint part 38a joining the distal end of the first wire 35a and the distal end of the second wire 35n, the distal end joint part 38b joining the distal end of the first wire 35b and the distal end of the second wire 35p, the distal end joint part 38c joining the distal end of the first wire 35c and the distal end of the second wire 35q, the distal end joint part 38d joining the distal end of the first wire 35d and the distal end of the second wire 35r, and the distal end joint part 38e joining the distal end of the first wire 35e and the distal end of the second wire 35s are each formed.

In the embodiment, in addition to the above-described five distal end joint parts 38a to 38e, seven joint parts 38f to 38m are further formed. That is, the joint part 38f joining the distal end of the second wire 35t to the first wire 35d, the joint part 38g joining the distal end of the second wire 35u to the first wire 35d, the joint part 38h joining the distal end of the second wire 35v to the first wire 35e, the joint part 38i joining the distal end of the second wire 35w to the first wire 35e, the joint part 38j joining the distal end of the second wire 35x to the first wire 35e, the joint part 38k joining the distal end of the second wire 35y to the first wire 35e, and the joint part 38m joining the distal end of the second wire 35z to the first wire 35e are each formed. In the embodiment, the second wires (35t to 35z) are joined to the same first wire 35e. However, each second wire may be joined to an arbitrary first wire. For example, a part or all of the second wires (35t to 35z) may be joined to mutually different first wires.

As illustrated in FIGs. 9 and 10, the distal end joint part 38a, the distal end joint part 38b, the distal end joint part 38c, the distal end joint part 38d, and the distal end joint part 38e are arranged at mutually different positions in the longitudinal direction of the catheter main body 33 on the same straight line (virtual straight line L4) parallel to the axial direction, similarly to the third embodiment.

Further, in the embodiment, the distal ends of all of the second wires are joined to the distal end of any of the first wires or to a point on the first wires other than the distal ends.

In FIG. 10, the first wire 35a and the second wire 35n do not exist on the more distal end side than the distal end joint part 38a. Thus, the rigidity on the more distal end side than the distal end joint part 38a is reduced as compared with the rigidity of the more proximal end side than the distal end joint part 38a, similarly to the first embodiment. Further, the distal end of the first wire 35a and the distal end of the second wire 35n are joined, which prevents fraying of the first wire 35a and the second wire 35n, similarly to the first embodiment. Furthermore, it is also possible to prevent fraying of other wires crossing the first wire 35a or the second wire 35n. The distal end joint part 38b, the distal end joint part 38c, the distal end joint part 38d, and the distal end joint part 38e also exert the same above-described effects as the distal end joint part 38a.

Further, in the embodiment, the distal ends of all of the second wires are joined to any point on the first wires, which prevents fraying of any of the second wires.

When examining the change in the longitudinal rigidity of the catheter main body 33, the rigidity of the catheter main body 33 is gradually reduced from the proximal end side toward the distal end side, similarly to the third embodiment. As compared with the third embodiment, it is recognized that, in the catheter 30 of the embodiment, the number of the wires is reduced toward the distal end because the number of distal end joint parts is increased, which increases the degree of reduction in rigidity.

In addition, the distal end joint part 38a, the distal end joint part 38b, the distal end joint part 38c, the distal end joint part 38d, and the distal end joint part 38e are arranged at different positions on the same straight line along the longitudinal direction of the catheter main body 33. This allows for easy manufacturing, similarly to the third embodiment.

Note that in FIG. 10, the distal end portions of the first wires (35f, 35g, 35h, 35i, 35j, 35k, and 35m) whose distal ends are not joined may extend to the distal end joint part 38a when only considering the rigidity of the catheter main body 33. However, when also considering fraying of the wires, the distal end portions of the first wires (35f, 35g, 35h, 35i, 35j, 35k, and 35m) may be located on the slightly more distal end side than the intersections with the second wires.

Note that the number of each of the first wires (35a to 35m) and the second wires (35n to 35z), the combination thereof, the wire width, the material, the cross-sectional shape, and the like may be changed variously as described in the first embodiment.

In the catheter 30 of the embodiment, the distal ends of all of the second wires (35n to 35z) are joined to the first wire. Thus, it is possible to further prevent fraying of the braid 35, alleviate a rigidity difference between the distal tip 2 and the braid 35, and suppress kinking of the catheter 30.

### (Fifth Embodiment)

Next, the fifth embodiment of the invention will be described. A catheter 40 of the fifth embodiment is different from the catheter 20 of the third embodiment in the processing method of the distal end of the first wire.

That is, in the first wires of the catheter 20 of the third embodiment, the distal ends of the four first wires (25a, 25b, 25c, and 25d) are respectively joined to the distal ends of the second wires (25n, 25p, 25q, and 25r), while in the second wires of the catheter 40 of the present embodiment, the distal ends of all of the first wires (45a, 45b, 45c, 45d, 45e, 45f, 45g, 45h, 45i, 45j, 45k, and 45m) are joined to distal ends of the second wires or to a point on the second wires other than the distal ends.

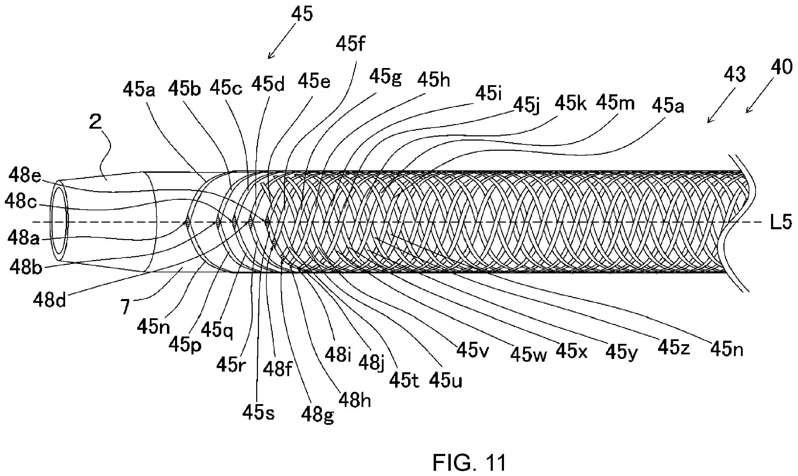

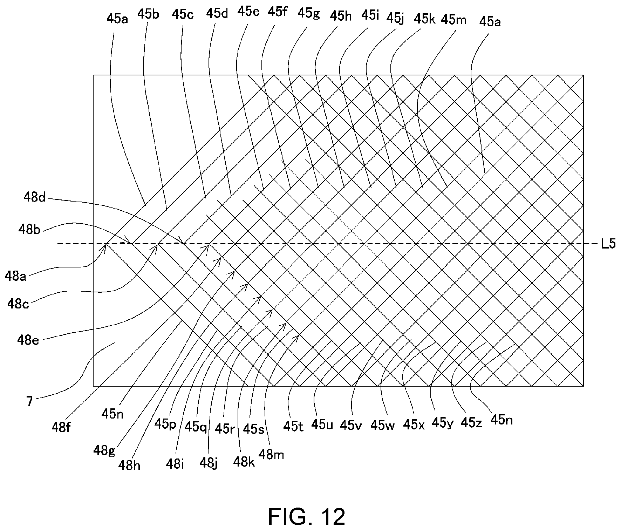

FIG. 11 is a perspective view of the distal end portion in which the outer layer is removed in the catheter according to the fifth embodiment and FIG. 12 is a developed view of the distal end portion in which the braid is developed on a plane surface in the catheter according to the fifth embodiment.

Note that the entire view of the catheter is the same as Fig. 1, and the longitudinal section view of the distal end portion of the catheter is also the same as Fig. 2. Thus, the description thereof is omitted. In the catheter 40 of the embodiment, a catheter main body 43 is different from the catheter main body 3 of the first embodiment. To be more specific, a braid 45 is an only difference from the braid 5 of the fourth embodiment, and other structures are the same as those of the first embodiment. Therefore, the same parts are denoted by the same reference numerals, and descriptions thereof may be omitted.

Similarly to the first embodiment, the braid 45 is formed of a total of 24 (12×12) wires including twelve first wires (45a, 45b, 45c, 45d, 45e, 45f, 45g, 45h, 45i, 45j, 45k, and 45m, 45a to 45m) and twelve second wires (45n, 45p, 45q, 45r, 45s, 45t, 45u, 45v, 45w, 45x, 45y and 45z, 45n to 45z) that are alternately woven into a mesh form.

In the embodiment, as illustrated in FIG. 11, the twelve first wires (45a to 45m) are wound counterclockwise from the proximal end toward the distal end of the catheter main body 43, and the twelve second wires (45n to 45z) are wound clockwise from the proximal end toward the distal end of the catheter main body 43.

The first wires (45a to 45m) and the second wires (45n to 45z) in the embodiment may be metal wires, similarly to the first embodiment, and may be stainless steel (SUS304 or SUS316), for example.

As illustrated in FIG. 12, the braid 45 of the embodiment includes five distal end joint parts 48a to 48e, similarly to the fourth embodiment.

In the embodiment, in addition to the above-described five distal end joint parts 48a to 48e, seven joint parts 48f to 48m are further formed, similarly to the fourth embodiment. That is, the joint part 48f joining the distal end of the first wire 45f to the second wire 45s, the joint part 48g joining the distal end of the first wire 45g to the second wire 45s, the joint part 48h joining the distal end of the first wire 45h to the second wire 45s, the joint part 48i joining the distal end of the first wire 45i to the second wire 45s, the joint part 48j joining the distal end of the first wire 45j to the second wire 45s, the joint part 48k joining the distal end of the first wire 45k to the second wire 45s, and the joint part 48m joining the distal end of the first wire 45m to the second wire 45s are each formed. In the embodiment, the first wires (45f to 45m) are joined to the same second wire 45s. However, each first wire may be joined to an arbitrary second wire. For example, a part or all of the first wires (45f to 45m) may be joined to mutually different second wires.

Similarly to the fourth embodiment, the distal end joint part 48a, the distal end joint part 48b, the distal end joint part 48c, the distal end joint part 48d, and the distal end joint part 48e are arranged at mutually different positions in the longitudinal direction of the catheter main body 43 on the same straight line (virtual straight line L5, see FIG. 11 and FIG. 12) parallel to the axial direction.

Further, in the embodiment, the distal ends of all of the first wires are joined to distal ends of the second wires or to a point on the second wires other than the distal ends.

As is understood from the above description, in the present embodiment, the distal end joint parts 48a to 48e are the same as in the fourth embodiment. Meanwhile, at the joint parts 48f to 48m, the distal ends of all of the first wires are joined to any of the second wires, and as compared with the fourth embodiment in which the distal ends of all of the second wires are joined to any one of the first wires, the first wires and the second wires are switched. Therefore, the embodiment is the same as the fourth embodiment in that the number of wires forming the braid 45 is reduced toward the distal end of the catheter main body 43. Further, regarding the prevention of fraying of wires forming the braid 45, the embodiment exerts the same effects even though the distal ends of all of the first wires are joined to the second wire and the individual wires prevented from fraying are different from the fourth embodiment.

Note that the number of each of the first wires (45a to 45m) and the second wires (45n to 45z), the combination thereof, the wire width, the material, the cross-sectional shape, and the like may be changed variously as described in the first embodiment.

In the catheter 40 of the embodiment, the distal ends of all of the first wires (45a to 45m) are joined to the distal ends of the second wires or to the second wires. Thus, it is possible to further prevent fraying of the braid 45, alleviate a rigidity difference between the distal tip 2 and the braid 45, and suppress kinking of the catheter 40.

Note that it is possible to combine the fourth embodiment and the fifth embodiment. That is, the first wire other than the first wires forming the distal end joint parts may be joined to any second wire, and the second wire other than the second wires forming the distal end joint parts may be joined to any first wire.

In all embodiments, it is possible to achieve a reduction of a rigidity difference between the distal tip of a catheter and a braid, in a catheter including a catheter main body having an inner layer, a braid wound around the outer periphery of the inner layer, and an outer layer covering the inner layer and the braid, along with a distal tip joined to the distal end of the catheter main body, wherein the braid includes first wires wound in one direction and second wires wound in a direction crossing the one direction, and, in the distal end portion of the braid, part of the first wires and the second wires are lacking.

### REFERENCE SIGNS LIST

- 1, 10, 20, 30, 40, 50, 60: Catheter
- 2: Distal tip
- 3, 13, 23, 33, 43, 53, 63: Catheter main body
- 4: Connector
- 5, 15, 25, 35, 45, 55, 65: Braid
- 6: Lumen
- 7: Inner layer
- 8, 18, 28, 38: Distal end joint part
- 9: Outer layer

## Claims

1. A catheter comprising: a catheter main body including an inner layer, a braid wound around an outer periphery of the inner layer, and an outer layer covering the inner layer and the braid; and
a distal tip joined to a distal end of the catheter main body, wherein
the braid includes a plurality of first wires wound in one direction and a plurality of second wires wound in a direction crossing the one direction, and
a first distal end joint part joining a distal end of the first first wire and a distal end of the first second wire and a second distal end joint part joining a distal end of the second first wire and a distal end of the second second wire are arranged at different positions in a longitudinal direction of the catheter main body.

2. The catheter according to claim 1, wherein the second distal end joint part is arranged on a straight line passing the first distal end joint part and being parallel to an axial direction of the catheter.

3. The catheter according to claim 1, wherein the second distal end joint part is arranged at a position separated from a straight line passing the first distal end joint part and being parallel to an axial direction of the catheter.

4. The catheter according to claim 3, wherein a third distal end joint part joining a distal end of the third first wire and a distal end of the third second wire is arranged at a different position in the longitudinal direction of the catheter main body than the first distal end joint part and the second distal end joint part, and at a position separated from both the straight line passing the first distal end joint part and being parallel to the axial direction of the catheter and a straight line passing the second distal end joint part and being parallel to the axial direction of the catheter.

5. The catheter according to any one of claims 1 to 4, wherein the distal ends of all of the first wires are joined to the distal end of any of the second wires or to a point on any second wire other than the distal end.

6. The catheter according to any one of claims 1 to 4, wherein the distal ends of all of the second wires are joined to the distal end of any of the first wires or to a point on any first wire other than the distal end.
